# EUROPEAN PATENT SPECIFICATION

(11) **EP 0 890 636 B1**
(45) Date of publication and mention of the grant of the patent: **31.10.2001**
(21) Application number: 98112851.5
(22) Date of filing: 10.07.1998
(51) Int. Cl.: C12M 3/06

(54) **Culture vessel**
Kulturgefäss
Récipient de culture

(30) Priority: 11.07.1997 JP 18629397
(43) Date of publication of application: 13.01.1999
(73) Proprietor: Menicon Co., Ltd., Nagoya-shi Aichi-ken (JP)
(72) Inventor: Sugiyama, Akihisa, Kasugai-shi, Aichi-ken (JP); Kamiya, Naotaka, Kasugai-shi, Aichi-ken (JP)
(74) Representative: Leson, Thomas Johannes Alois, Dipl.-Ing.

(56) References cited:
- DE-C- 19 512 531
- US-A- 4 435 508
- DATABASE WPI Section Ch, Week 9635 Derwent Publications Ltd., London, GB; Class A89, AN 96-344456 XP002081450 & JP 06 335385 A (SUMITOMO BAKELITE CO LTD), 6 December 1994
- 5th General meeting of ESACT, Copenhagen, Denmark, 1982, Develop. Biol. Standard vol. 55, pp. 81-92, R. E. Spier & B. Griffiths.
- Nihon Univ. Dent. J., vol. 62, pp. 8-19, 1988.

## Description

The present invention relates to a culture vessel, and more particularly to a culture vessel which can be constantly and sufficiently supplied with gas such as oxygen necessary for metabolism of cells without an oxygenator, and the like, and which is not contaminated with bacteria and is easily transported.

When cells are usually cultured, it is essential to supply oxygen necessary for metabolism of cells. As a culture vessel a flask for cell culture, Petri dish, and the like have been conventionally used. When the flask is used in cell culture, aeration is generally kept by loosening a cap thereof in order to supply oxygen, and carbonic acid gas of about 5 % which is used to decrease a pH change of medium due to metabolism of cells. When Petri dish is used in cell culture, aeration is kept by designing a cap so as to form a small clearance between the cap and the body when the cap is put on the body.

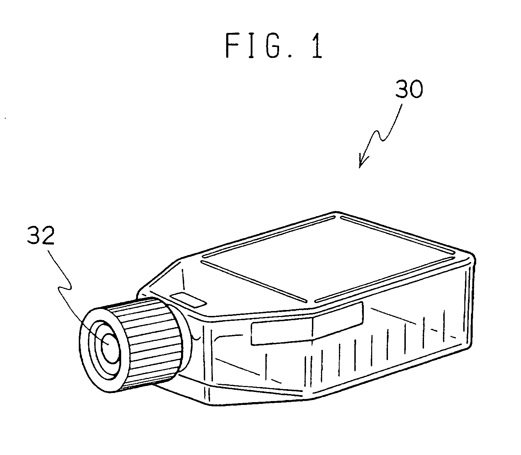

However, those cases have serious disadvantage, that bacterial contamination often occurrs. FALCON® Cell Culture Flask commercially available from Collaborative Biomedica Products is a flask for cell culture 30, as shown in Fig. 1 of the present specification, wherein a filter made of polyethylene fiber 32 is used in order to avoid bacterial contamination and to keep aeration.

On the other hand, it is conventionally almost impossible to culture cells in a hermetic culture system, namely, when gas cannot be added into a vessel because the vessel is filled with medium, the cells cannot almost be cultured in the vessel. There is also a disadvantage that a vessel has to be transported in the shortest possible time, when the vessel filled with medium is transported.

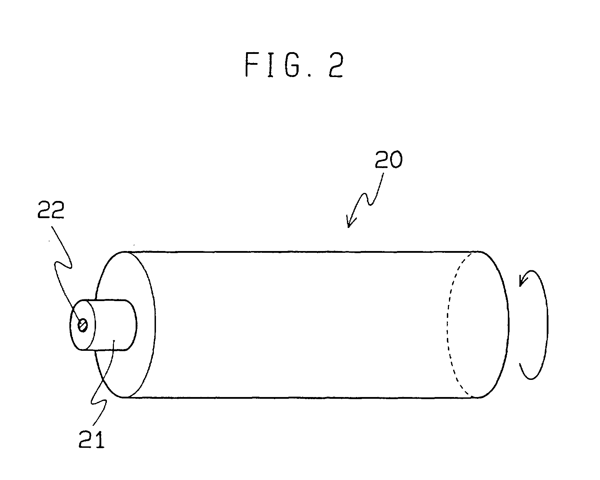

FALCON® Roller Bottle commercially available from Collaborative Biomedica Products is a vessel for large-scale culture using a rollar bottle 20, as shown in Fig. 2 in the present specification. The large-scale culture using the vessel has, however, inconveniences that a medium adheres to and seeps through a filter 22 equipped on a cap 21 for exchanging gas, and that bacterial contamination occurs therefrom.

Furthermore, when cells are cultured in large-scale, an oxygenator for supplying oxygen sufficiently is needed.

Japanese Unexamined Patent Publication No. 49364/1993 discloses a vessel for hatching a fertilized egg wherein at least a part of the body or cap of the vessel is made of gas permeable material which is formed by polymerizing a monomer mixture mainly comprising tris(trimethylsiloxy)silylstyrene. However, the bottom surface of the vessel is necessary to be a curved surface, and then, the vessel for hatching has a defect in that it cannot be easily transported.

Japanese Unexamined Patent Publication No. 233776/1988 discloses a culture vessel wherein plural hollow fibers having gas permeability are used as a bed for culture. An object of the culture vessel is to culture cells in large-scale and high density. However, the vessel has some disadvantages such that a process for culture is complicate because plural hollow fibers are used, that a medium seeps out, and that bacterial contamination occurs therefrom.

US-A-4,435,508 discloses a tissue culture vessel comprising a growth surface which may be a sheet of a gas-permeable membrane made of Teflon®. The disclosed tissue culture vessel permits ready sterile access to perform manipulative operations on tissue cultures growing therein.

DE-C-195 12 531 discloses a culture vessel for cell cultures comprising a gas-permeable gas transfer membrane made of a silicone molding member. The construction of the disclosed culture vessel guarantees a sufficient supply of nutrients and oxygen to the cell cultures and disposal of cell metabolies.

JP-A-6 335 385 discloses a culture vessel for the freeze storage of cells equipped with a transparent rubber-like coating which may serve as a culture surface on a main body of the vessel. Silicone is mentioned as the preferred coating. For providing hydrophilicity, the surface of the coating may be subjected to e.g. corona discharge, a low-temperature plasma treatment or UV irradiation.

In view of the foregoing, it is an object of the present invention is to provide a culture vessel which can be constantly and sufficiently supplied with gas such as oxygen necessary for metabolism of cells without the oxygenator, and the like.

A further object of the invention is to provide a culture vessel which is not contaminated by bacteria and is easily transported.

Those and other objects of the invention will become apparent from the description hereinafter.

It has been found that a vessel, at least a part of which comprises a gas permeable plastic material is useful for cell culture.

In accordance with the present invention, there is provided a culture vessel wherein at least a part of the vessel is made of a gas permeable plastic material, as defined in claim 1.

In accordance with the invention, it is possible to constantly supply gas such as oxygen, and the like necessary for metabolism of cells into the vessel, thereby being also possible to culture cells even if the vessel is hermetically sealed. Furthermore, the vessel is also easily transported because the cells can be maintained for long time in the vessel filled with the medium.
Fig. 1 is an explanatory view of a conventional cell culture vessel.
Fig. 2 is an explanatory view of a conventional vessel for cell mass culture.
Fig. 3 is an explanatory view schematically showing one embodiment of the cell culture vessel of the present invention.
Fig. 4 is an explanatory view schematically showing one embodiment of the cell culture vessel of the present invention.

The culture vessel of the present invention is a vessel wherein at least a part of the vessel is made of a gas permeable plastic material.

The term "gas permeable plastic material" as herein used means a high-molecular material of which oxygen permeability is at least 1 × 10⁻¹¹ mℓ (O₂)·cm/(cm²·sec mmHg).

As the gas permeable plastic material, for example, a contact lens material can be preferably employed because it has high oxygen permeability and has high strength. The gas permeable plastic material is a polymer obtainable by polymerizing a monomer mixture containing a silicon-containing monomer selected from a silicon-containing (meth)acrylate, a silicon-containing styrene derivative, and a silicon-containing urethane macromonomer.

Further, the gas permeable plastic material preferably has transparency to the extent that cells in the vessel can be observed.

The term "...(meth)acrylate" as herein used means "...acrylate and/or ...methacrylate". Also, the term "...(meth)acrylate derivative" as herein used means "...acrylate derivative and/or ...methacrylate derivative ".

Examples of the silicon-containing monomer are, for instance, a silicon-containing urethane macromonomer, and the like as well as the silicon-containing (meth)acrylate and the silicon-containing styrene derivative. Those may be used alone or in admixture thereof.

As the silicon-containing (meth)acrylate, there can be preferably employed, for instance, a silicon-containing (meth)acrylate represented by the formula (I): 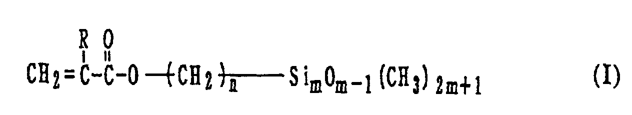 wherein R is hydrogen atom or methyl group, n is an integer of 1 to 3 and m is an integer of 1 to 4.

Examples of the silicon-containing (meth)acrylate are, for instance, trimethylsiloxydimethylsilylmethyl (meth)acrylate, trimethylsiloxydimethylsilylpropyl (meth)acrylate, methylbis(trimethylsiloxy)silylpropyl (meth)acrylate, tris(trimethylsiloxy)silylpropyl (meth)acrylate, mono[ methylbis(trimethylsiloxy)siloxy]bis(trimethylsiloxy)silylpropyl (meth)acrylate, tris[methylbis(trimethylsiloxy)siloxy]silylpropyl (meth)acrylate, methylbis(trimethylsiloxy)silylpropylglyceryl (meth)acrylate, tris(trimethylsiloxy)silylpropylglyceryl (meth)acrylate, mono[ methylbis(trimethylsiloxy)siloxy]bis(trimethylsiloxy)silylpropylglyceryl (meth)acrylate, trimethylsilylethyltetramethyldisiloxypropylglyceryl (meth)acrylate, trimethylsilylmethyl (meth)acrylate, trimethylsilylpropyl (meth)acrylate, trimethylsilylpropylglyceryl (meth)acrylate, trimethylsiloxydimethylsilylpropylglyceryl (meth)acrylate, methylbis(trimethylsiloxy)silylethyltetramethyldisiloxymethyl (meth)acrylate, tetramethyltriisopropylcyclotetrasiloxanylpropyl (meth)acrylate, tetramethyltripropylcyclotetrasiloxybis(trimethylsiloxy)silylpropyl (meth)acrylate, and the like. Those may be used alone or in admixture thereof.

As the silicon-containing styrene derivative, there can be preferably employed, for instance, a silicon-containing styrene derivative represented by the formula (II): 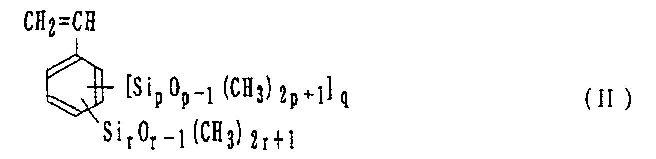 wherein p is an integer of 1 to 15, q is 0 or 1 and r is an integer of 1 to 15, with a proviso that a case of q=0 and r=1 is excepted.

Examples of the silicon-containing styrene derivative represented by the formula (II) are, for instance, tris(trimethylsiloxy)silylstyrene, bis(trimethylsiloxy)methylsilylstyrene, (trimethylsiloxy)dimethylsilylstyrene, tris(trimethylsiloxy)siloxydimethylsilylstyrene, [bis(trimethylsiloxy)methylsiloxy]dimethylsilylstyrene, heptamethyltrisiloxanylstyrene, nonamethyltetrasiloxanylstyrene, pentadecamethylheptasiloxanylstyrene, heneicosamethyldecasiloxanylstyrene, heptacosamethyltridecasiloxanylstyrene, trimethylsiloxypentamethyldisiloxymethylsilylstyrene, tris(pentamethyldisiloxy)silylstyrene, tris(trimethylsiloxy)siloxybis(trimethylsiloxy)silylstyrene, bis(heptamethyltrisiloxy)methylsilylstyrene, tris[methylbis(trimethylsiloxy)siloxy]silylstyrene, trimethylsiloxybis[tris(trimethylsiloxy)siloxy]silylstyrene, nonamethyltetrasiloxyundecylmethylpentasiloxymethylsilylstyrene, tris[ tris(trimethylsiloxy)siloxy]silylstyrene, tris(trimethylsiloxyhexamethyl)tetrasiloxy-tris(trimethylsiloxy)siloxytrimethylsiloxysilylstyrene, nonakis(trimethylsiloxy)tetrasiloxanylstyrene, bis(tridecamethylhexasiloxy)methylsilylstyrene, and the like. Those may be used alone or in admixture thereof.

Examples of the silicon-containing urethane macromonomer are, for instance, a polysiloxane macromonomer in which a polymerizable group bonds to a siloxane main chain through one or two urethane bond(s), and which is represented by the formula:

A¹-U¹-S¹-U²-A²

[wherein A¹ is a group represented by the formula:

Y²¹-R³¹-

in which Y²¹ is acryloyloxy group, methacryloyloxy group, vinyl group or allyl group and R³¹ is a straight-chain or branched alkylene group having 2 to 6 carbon atoms;
A² is a group represented by the formula:

   -R³⁴-Y²²

   in which Y²² is acryloyloxy group, methacryloyloxy group, vinyl group or allyl group and R³⁴ is a straight-chain or branched alkylene group having 2 to 6 carbon atoms;
U¹ is a group represented by the formula:

   -X²¹-E²¹-X²⁵-R³²-

   in which X²¹ is a covalent bond, oxygen atom or an alkylene glycol group having 1 to 6 carbon atoms, E²¹ is -CONH- group (in this case, X²¹ is a covalent bond and an urethane bond is formed between E²¹ and X²⁵) or a divalent group derived from a diisocyanate selected from a group consisting of a saturated or unsaturated aliphatic diisocyanate, an alicyclic diisocyanate and an aromatic diisocyanate (in this case, X²¹ is oxygen atom or an alkylene glycol group having 1 to 6 carbon atoms and E²¹ forms an urethane bond between X²¹ and X²⁵), X²⁵ is oxygen atom or an alkylene glycol group having 1 to 6 carbon atoms, and R³² is a straight-chain or branched alkylene group having 1 to 6 carbon atoms;
S¹ is a group represented by the formula: 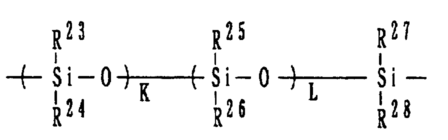 in which each of R²³, R²⁴, R²⁵, R²⁶, R²⁷ and R²⁸ is independently an alkyl group having 1 to 6 carbon atoms, an alkyl group substituted with fluorine or phenyl group. K is an integer of 1 to 50, L is an integer of 0 to (50-K); and
U² is a group represented by the formula:

   -R³³-X²⁶-E²²-X²²-

   in which R³³ is a straight-chain or branched alkylene group having 1 to 6 carbon atoms, X²² is a covalent bond, oxygen atom or an alkylene glycol group having 1 to 6 carbon atoms, X²⁶ is oxygen atom or an alkylene glycol group having 1 to 6 carbon atoms, and E²² is -CONH- group (in this case, X²² is a covalent bond and an urethane bond is formed between E²² and X²⁶) or a divalent group derived from a diisocyanate selected from a group consisting of a saturated or unsaturated aliphatic diisocyanate, an alicyclic diisocyanate and an aromatic diisocyanate (in this case, X²² is oxygen atom or an alkylene glycol group having 1 to 6 carbon atoms and E²² forms an urethane bond between X²² and X²⁶)], such as urethane bond-containing polysiloxane macromonomer represented by the formula (III): 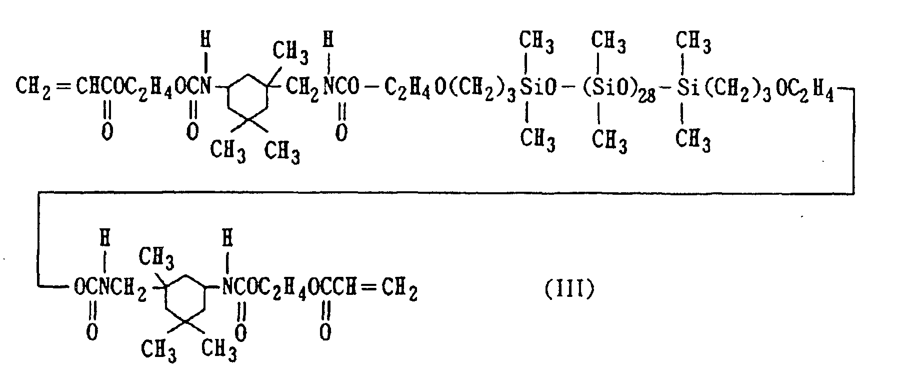 , or polysiloxane macromonomer represented by the formula: 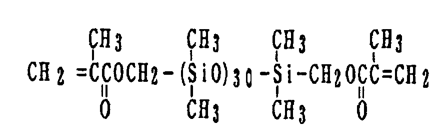
and the like.

It is desired that the amount of the silicon-containing monomer is adjusted to at least 1 part by weight, preferably at least 5 parts by weight, based on 100 parts by weight of the total amount of the monomer mixture in order to give high gas permeability to the materials. Also, it is desired that the amount of the silicon-containing monomer is adjusted to at most 99.9 parts by weight, preferably at most 95 parts by weight, based on 100 parts by weight of the total amount of the monomer mixture in order to give high strength to the materials.

Furthermore, the monomer mixture used for preparing the gas permeable plastic material may include, for instance, a polymerizable monomer usually used for contact lens materials, such as a fluorine-containing monomer, a monomer for adjusting hardness, a hydrophilic monomer and a crosslinkable monomer, as well as the above silicon-containing monomer.

As the fluorine-containing monomer, there can be employed, for instance, a fluorine-containing (meth)acrylate, a fluorine-containing styrene derivative, and the like.

Examples of the fluorine-containing (meth)acrylate are, for instance, trifluoromethyl (meth)acrylate, 2,2, 2-trifluoroethyl (meth)acrylate, 2,2,3,3-tetrafluoropropyl (meth)acrylate, 2,2,3,3-tetrafluoro-t-pentyl (meth)acrylate, 2,2,3,4,4,4-hexafluorobutyl (meth)acrylate, 2,2,3,4,4,4-hexafluoro-t-hexyl (meth)acrylate, 2,3,4,5,5,5-hexafluoro-2, 4-bis(trifluoromethyl)pentyl (meth)acrylate, 2,2,3,3,4,4-hexafluorobutyl (meth)acrylate, 2,2,2,2',2',2 '-hexafluoroisopropyl (meth)acrylate, 2,2,3,3,4,4,4-heptafluorobutyl (meth)acrylate, 2,2,3,3,4,4,5,5-octafluoropentyl (meth)acrylate, 2, 2, 3,3,4,4,5,5, 5-nonafluoropentyl (meth)acrylate, 2,2,3,3,4,4,5,5,6,6,7,7-dodecafluoroheptyl (meth)acrylate, 3,3,4,4,5,5,6,6,7,7,8,8-dodecafluorooctyl (meth)acrylate, 3,3,4,4,5,5,6,6,7,7,8,8, 8-tridecafluorooctyl (meth)acrylate, 2,2,3,3,4,4,5,5,6,6,7,7,7-tridecafluoroheptyl (meth)acrylate, 3,3,4,4,5,5,6,6,7,7,8,8,9,9,10,10-hexadecafluorodecyl (meth)acrylate, 3,3,4,4,5,5,6,6,7,7,8,8,9,9,10,10,10-heptadecafluorodecyl (meth)acrylate, 3,3,4,4,5,5,6,6,7,7,8,8,9,9,10,10,11,11-octadecafluoroundecyl (meth)acrylate, 3,3,4,4,5,5,6,6,7,7,8,8,9,9,10,10,11,11,11-nonadecafluoroundecyl (meth)acrylate, 3,3,4,4,5,5,6,6,7,7,8,8,9,9,10,10,11,11,12,12-eicosafluorododecyl (meth)acrylate, and the like. Those may be used alone or in admixture thereof.

Examples of the fluorine-containing styrene derivative are, for instance, 2,2,2-trifluoroethylstyrene, 2,2,3,3-tetrafluoropropylstyrene, 2,2,3,3-tetrafluoro-t-pentylstyrene, 2,2,3,4,4,4-hexafluorobutylstyrene, 2,2,3,4,4,4-hexafluoro-t-hexylstyrene, 2,2,3,3,4,4-hexafluorobutylstyrene, 2,2,2,2',2',2'-hexafluoroisopropylstyrene, 2,2,3,3,4,4,4-heptafluorobutylstyrene, 2,2,3,3,4,4,5,5-octafluoropentylstyrene, 2,2,3,3,4,4,5,5,5-nonafluoropentylstyrene, 4-vinylbenzyl-2 ',2',2'-trifluoroethyl ether, 4-vinylbenzyl-3',3',3'-trifluoropropyl ether, 4-vinylbenzyl-4 ',4',4'-trifluorobutyl ether, 4-vinylbenzyl-2',2',3',3',3'-pentafluoropropyl ether, 4-vinylbenzyl-2',2',3',3',4',4',4'-heptafluorobutyl ether, 4-vinylbenzyl-3',3',4',4',5',5',6',6',6'-nonafluorohexyl ether, 4-vinylbenzyl-3',3',4',4',5',5',6',6',7',7',8',8',9',9',-10',10',10'-heptadecafluorodecyl ether, and the like. Those can be used alone or in admixture thereof.

As the monomer for adjusting hardness, there can be employed, for instance, an alkyl (meth)acrylate, an alkylstyrene derivative, styrene, and the like.

Examples of the alkyl (meth)acrylate are, for instance, straight-chain, branched or cyclic alkyl (meth)acrylates such as methyl (meth)acrylate, ethyl (meth)acrylate, isopropyl (meth)acrylate, n-propyl (meth)acrylate, isobutyl (meth)acrylate, n-butyl (meth)acrylate, 2-ethylhexyl (meth)acrylate, n-octyl (meth)acrylate, n-decyl (meth)acrylate, n-dodecyl (meth)acrylate, t-butyl (meth)acrylate, n-pentyl (meth)acrylate, t-pentyl (meth)acrylate, hexyl (meth)acrylate, heptyl (meth)acrylate, nonyl (meth)acrylate, stearyl (meth)acrylate, cyclopentyl (meth)acrylate and cyclohexyl (meth)acrylate; alkoxyalkyl (meth)acrylates such as 2-ethoxyethyl (meth)acrylate, 3-ethoxypropyl (meth)acrylate, 2-methoxyethyl (meth)acrylate and 3-methoxypropyl (meth)acrylate; alkylthioalkyl (meth)acrylates such as ethylthioethyl (meth)acrylate and methylthioethyl (meth)acrylate; and the like.

Examples of the alkylstyrene derivative are, for instance, α -methylstyrene; alkylstyrenes such as methylstyrene, ethylstyrene, propylstyrene, n-butylstyrene, t-butylstyrene, isobutylstyrene and pentylstyrene; alkyl- α -methylstyrenes such as methyl- α -methylstyrene, ethyl- α -methylstyrene, propyl- α -methylstyrene, n-butyl- α -methylstyrene, t-butyl- α -methylstyrene, isobutyl- α -methylstyrene and pentyl- α -methylstyrene; and the like.

As the hydrophilic monomer, there can be employed, for instance, monomers having hydroxyl group, amido group, carboxyl group, amino group, glycol residue, pyrrolidone skeleton, and the like.

Examples of the hydrophilic monomer are, for instance, hydroxyl group-containing (meth)acrylates such as hydroxyethyl (meth)acrylate, hydroxypropyl (meth)acrylate, hydroxybutyl (meth)acrylate, dihydroxypropyl (meth)acrylate, dihydroxybutyl (meth)acrylate, diethylene glycol mono(meth)acrylate, triethylene glycol mono(meth)acrylate, propylene glycol mono(meth)acrylate and dipropylene glycol mono(meth)acrylate; (meth)acrylic acid; (meth)acrylamide monomers such as (meth)acrylamide, N-methyl(meth)acrylamide, N-ethyl(meth)acrylamide, N, N-dimethyl(meth)acrylamide, N, N-diethyl(meth)acrylamide and N, N-methylethyl(meth)acrylamide; vinyllactams such as N-vinylpyrrolidone, N-vinylpiperidone, N-vinylcaprolactam and N-vinylcapryllactam; aminoalkyl (meth)acrylates such as aminoethyl (meth)acrylate, N-methylaminoethyl (meth)acrylate, N, N-dimethylaminoethyl (meth)acrylate and 2-butylaminoethyl (meth)acrylate; alkoxy group-containing (meth)acrylates such as methoxyethyl (meth)acrylate, ethoxyethyl (meth)acrylate and methoxydiethylene glycol (meth)acrylate; maleic anhydride; maleic acid; fumaric acid; fumaric acid derivatives; aminostyrene; hydroxystyrene; and the like. Those may be used alone or in admixture thereof.

Examples of the crosslinkable monomer are, for instance, ethylene glycol di(meth)acrylate, diethylene glycol di(meth)acrylate, triethylene glycol di(meth)acrylate, propylene glycol di(meth)acrylate, dipropylene glycol di(meth)acrylate, diallyl fumarate, allyl (meth)acrylate, vinyl (meth)acrylate, trimethylolpropane tri(meth)acrylate, methacryloyloxyethyl (meth)acrylate, divinylbenzene, diallyl phthalate, diallyl adipate, triallyl diisocyanate, α -methylene-N-vinylpyrrolidone, 4-vinylbenzyl (meth)acrylate, 3-vinylbenzyl (meth)acrylate, 2,2-bis((meth)acryloyloxyphenyl)hexafluoropropane, 2, 2-bis((meth)acryloyloxyphenyl)propane, 1,4-bis(2-(meth)acryloyloxyhexafluoroisopropyl)benzene, 1,3-bis(2-(meth)acryloyloxyhexafluoroisopropyl)benzene, 1,2-bis(2-(meth)acryloyloxyhexafluoroisopropyl)benzene, 1,4-bis(2-(meth)acryloyloxyisopropyl)benzene, 1,3-bis(2-(meth)acryloyloxyisopropyl)benzene, 1,2-bis(2-(meth)acryloyloxyisopropyl)benzene, and the like. Those may be used alone or in admixture thereof.

The amount of the polymerizable monomers may be suitably adjusted so that the total amount of the monomer mixture of the polymerizable monomers and other monomers such as the silicon-containing monomer gives the amount of 100 parts by weight. Namely, it is desired that the amount of the polymerizable monomers is adjusted to at least 0.1 part by weight, preferably at least 0.05 part by weight, based on 100 parts by weight of the total amount of the monomer mixture. Also, it is desired that the amount of the polymerizable monomers is adjusted to at most 99 parts by weight, preferably at most 95 parts by weight, based on 100 parts by weight of the total amount of the monomer mixture.

Among the polymerizable monomers, when the crosslinkable monomer is used, it is desired that the amount of the crosslinkable monomer is adjusted to at least 0.01 part by weight, preferably at least 0.05 part by weight, based on 100 parts by weight of the total amount of the monomer mixture in order to sufficiently exhibit effects for improving mechanical strength and for imparting durability. Also, it is desired that the amount of the crosslinkable monomer is adjusted to at most 10 parts by weight, preferably at most 5 parts by weight, based on 100 parts by weight of the total amount of the monomer mixture in order to obviate a danger such that the gas permeable plastic material becomes brittle.

When the monomer mixture is polymerized in order to prepare the polymer constituting the gas permeable plastic material, usual polymerization methods can be employed. As the methods, there can be employed, for instance, a method comprising adding a radical polymerization initiator to the monomer mixture and heating little by little the resulting mixture from the room temperature to about 130°C, a method comprising irradiating the monomer mixture with an electromagnetic wave such as microwave, ultraviolet rays or radiation (gamma rays), and the like. When the polymerization is carried out by a thermal polymerization, the polymerization temperature can be raised stepwise. The polymerization can be carried out by a bulk polymerization method, a solution polymerization method using a solvent, and the like, or other methods.

Examples of the radical polymerization initiator are, for instance, azobisisobutyronitrile (hereinafter referred to as AIBN), azobisdimethylvaleronitrile, benzoyl peroxide, t-butyl hydroperoxide, cumene hydroperoxide, and the like. Those may be used alone or in admixture thereof. When the polymerization is carried out utilizing a ray, and the like, it is desired that a photopolymerization initiator and a sensitizer are further added to the monomer mixture. It is desired that the amount of the above polymerization initiator and the sensitizer is 0.002 to 2 parts by weight, preferably 0.01 to 1 part by weight, based on 100 parts by weight of the total amount of the monomer mixture.

Such polymer prepared as described above is molded into the gas permeable plastic material which constitutes at least a part of the culture vessel.

A molding method of the gas permeable plastic material is not particularly limited and usual molding methods carried out by a person skilled in the art can be employed. As the above molding methods, there can be employed, for instance, a cutting process, and the like. The cutting process is a method comprising carrying out the polymerization as described above in a suitable mold or a suitable container to give a material (polymer), for instance, in the form of bar, block, plate, or the like and subjecting the polymer to a mechanical process such as the cutting process to give a molded article having a desired shape.

The gas permeable plastic material made as described above can be in any form, preferably in the form of film in view of the gas permeability.

For instance, when the gas permeable plastic material is in the form of film, it is desired that thickness of the film is at least 0.01 mm, preferably at least 0.1 mm in order to impart strength to the gas permeable plastic material. Also, it is desired that thickness of the film is at most 50 mm, preferably at most 30 mm in order to impart suitable gas permeability to the plastic material.

It is desired that the gas permeable plastic material is nonporous.

The term "porous" as herein used means a state where physically and spatially defined pores exist in the plastic material. The term "nonporous" as herein used means a state where statistical void space (free volume) produced by movement (vibration) of high-molecular chain exists, but the above-mentioned physically and spatially defined pores do not exist. Concretely, the nonporous plastic material has an effect that gas permeates the plastic material by dissolution and diffusion of gas.

The porous plastic material is not desired because it is opaque due to pores thereof.

Furthermore, the gas permeable plastic material of which surface has hydrophilic property is subjected to a plasma treatment to render it hydrophilic in order to impart suitable adhesiveness of the cells thereto at the cell culture. As the above method for imparting hydrophilic property, there is employed a plasma treatment, which method may optionally combined with any of the following methods: a method comprising immersing the gas permeable plastic material in a solution for imparting hydrophilic property, a method comprising subjecting the gas permeable plastic material to a chemical reaction. The plasma treatment imparts excellent hydrophilic property and durability to the gas permeable plastic material.

When the gas permeable plastic material possesses hydrophilic property in the present invention, the gas permeable plastic material can be a polymer obtained by polymerizing the monomer mixture, which is not processed into the desired form such as bar, block or plate, or can be a polymer in a form obtained by the cutting process, and the like from the polymer, for instance, in the form of film.

It is desired that the above plasma treatment is usually carried out in a gaseous atmosphere, for instance, oxygen, hydrogen, nitrogen, argon, helium, ammonia, carbon monoxide, carbon dioxide, methane, ethane or propane atmosphere under the pressure of 0.1 to 1 torr.

As the apparatus for the plasma treatment, it is desired that a vacuum chamber equipped with an oscillator for high frequency, low frequency, and the like is used. Particularly, the treatment utilizing high frequency is desired, and a high frequency of 13.56 MHz is usually used. Also, it is desired that the ordinal power is of 10 to 55 W and the treatment time is of 3 seconds to 5 minutes.

As the method comprising immersing the gas permeable plastic material in a solution for imparting hydrophilic property, it is desired to employ a method such as a method comprising coating the gas permeable plastic material with a sulfuric acid solution containing potassium bichromate, a dilute aqueous solution containing collagen or an aqueous solution containing heparine by immersing the material therein; a method of treatment for imparting hydrophilic property which is characterized by immersing the gas permeable plastic material in an aqueous solution containing a hydrophilic polymer having a photoreactive group described in Japanese Unexamined Patent Publication No. 136867/1996, followed by irradiating the material with rays through the aqueous solution; and a method of acid treatment comprising immersing the gas permeable plastic material in various organic acids such as acetic acid, formic acid and trifluoroacetic acid; or inorganic acids such as hydrochloric acid, sulfuric acid and phosphoric acid. Also, after immersion the plasma treatment can be carried out.

As the method comprising subjecting the gas permeable plastic material to a chemical reaction, it is desired to employ a method for imparting hydrophilic property comprising subjecting the plastic material to a reaction with a methoxysilane having amino group, for instance, such as aminopropylmethoxysilane (commercially available under the trademark LS-1420 from Shin-Etsu Chemical Co., Ltd., JAPAN).

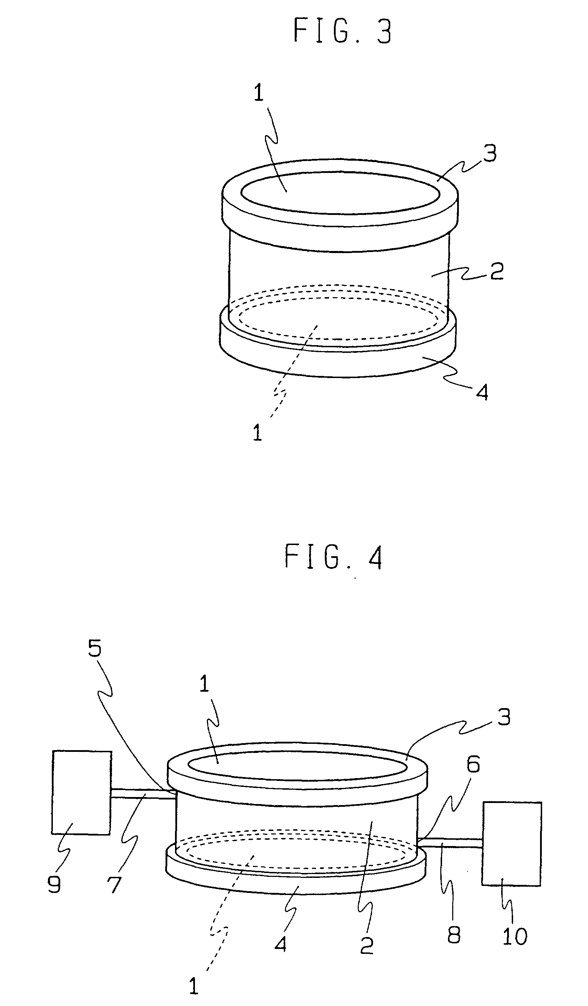

In accordance with the present invention, the gas permeable plastic material can be used in the body of the vessel or the whole culture vessel. Furthermore, the gas permeable plastic material can be used in a surface for adhesion of cultured cells thereto. It is desired that, for instance, the gas permeable plastic material is used in a surface to which cells adhere to proliferate (for instance, the gas permeable plastic material film (1 in Fig. 3), and the like) when culture is monolayer culture, and the material is used in a surface for adhesion of suspended cells thereto (for instance, the body of the vessel 2 (2 in Fig. 3), the gas permeable plastic material film (1 in Fig. 3), and the like) when culture is suspension culture.

It is desired that the area wherein the gas permeable plastic material is provided is at least 1 % based on the total surface area of the culture vessel in order to sufficiently supply gas such as oxygen, or the like which is necessary for metabolism of cells. Also, it is desired that the area wherein the gas permeable plastic material is provided is at most 100 % based on the total surface area of the culture vessel in order suitably to culture cells in a large-scale.

When the gas permeable plastic material is not used in the whole culture vessel, a material constituting the part which is not made of the gas permeable plastic material is not particularly limited as far as the material is not denaturated by conditions of culture (pH of a medium, and the like), components of the material are not melted out, the material does not affect cell culture, and the material resists in sterilization. As the material constituting the part which is not made of the gas permeable plastic material, there can be employed, for instance, acrylic resin, polycarbonate, polypropylene, polystyrene, ABS resin, BS, TPX, and the like.

With referring to Fig. 3 and Fig. 4, an example of the culture vessel of the present invention will be explained in detail. Fig. 3 is an explanatory view schematically showing one embodiment of the culture vessel of the invention. Fig. 4 is an explanatory view schematically showing one embodiment of the culture vessel of the invention wherein a unit for supplying a medium 9 is connected to the culture vessel shown in Fig. 3 and a unit for discharging the medium and for collecting a supernatant of the medium or cells 10 is connected to the vessel.

The culture vessel of the invention can be employed in any structure as far as cells are suitably cultured in the vessel, preferably a hermetic structure. The term "hermetic structure" as herein used means a structure which allows sufficient aeration necessary for carrying out the usual cell culture, but does not allow seepage of an inside liquid of the vessel and does not allow contamination by an outside liquid of the vessel. The structure means, for instance, a structure which allow aeration of oxygen, carbonic acid gas, and the like, but does not allow seepage of the medium inside of the vessel and does not allow bacterial contamination.

The culture vessel of the invention is preferably a vessel, a bottom surface of which is not a curved surface.

The culture vessel shown in Fig. 3 comprises the gas permeable plastic material film 1, the body of the vessel 2, the top ring 3 and the bottom ring 4. The culture vessel shown in Fig. 4 comprises the supply port 5, the discharge port 6, the supply pipe 7, the discharge pipe 8, the unit for supplying a medium 9 and the unit for discharging a medium and for collecting a supernatant of the medium or cells 10, in addition to the gas permeable plastic material film 1, the body of the vessel 2, the top ring 3 and the bottom ring 4.

The body of the vessel 2, the top ring 3 and the bottom ring 4 can be made of, for instance, acrylic resin, polycarbonate, polypropylene, polystyrene, ABS resin, BS, TPX.

The setting of the gas permeable plastic material film 1 to the body of the vessel 2 can be carried out, for instance, by the procedure comprising covering the bottom opening of the body of the vessel 2 with the gas permeable plastic material film 1, fixing the gas permeable plastic material film 1 to the body of the vessel 2 using the bottom ring 4, pouring a suspension of the cells and a medium into the vessel, covering the top opening of the body of the vessel 2 with the gas permeable plastic material film 1 and fixing the gas permeable plastic material film 1 to the body of the vessel 2 using the top ring 3 to make the culture vessel hermetic.

Also, in at least a part of the culture vessel, the supply port 5 of the medium can be provided for exchanging of the medium.

When the supply port 5 is provided, the unit for supplying a medium 9 can be connected to the body of the vessel 2 via the supply pipe 7 as shown in Fig. 4.

The above unit for supplying a medium 9 comprises, for instance, a tube made of silicone, a filter made of cellulose acetate, a pump and a vessel containing the medium to be supplied.

Furthermore, in at least a part of the culture vessel, the discharge port 6 may be provided for discharging the medium or for collecting the supernatant of the medium or cells.

When the vessel of the invention is provided with the discharge port 6, the unit for discharging a medium and for collecting a supernatant of the medium or cells 10 can be connected to the body of the vessel 2 via the discharge pipe 8 as shown in Fig. 4.

The unit for discharging a medium and for collecting a supernatant of the medium or cells 10 comprises, for instance, a tube made of silicone, a filter made of cellulose acetate and a vessel for storing the discharged medium, the supernatant of the medium or cells.

The culture vessel of the invention can be used for a usual method of cell culture. As the method of cell culture, there can be employed, for instance, a method of monolayer culture, a method of stationary culture or shaking culture of suspended cells, a method of semisolid culture, a method of large-scale culture, a method of multilayer culture, and the like.

Examples of the culture vessel for large-scale culture of the invention are, for instance, a rollar bottle, and the like. Such rollar bottle may be made of the gas permeable plastic material in at least a part or the whole of the bottle, so that it is possible to sufficiently supply oxygen without a conventional cap equipped with a membrane filter and bacterial contamination therefrom is out of anxiety. In addition, by employing such rollar bottle it is not necessary to equip an apparatus for supplying gas being necessary for culturing large-scale cells, so that cells can also be cultured inexpensively and easily in large-scale.

The cells cultured may be usual animal cells or plant cells. Examples of the cultured cells, for instance, fibroblasts (3T3 cells, and the like), epidermal cells or fibroblasts derived from mammalian skin, melanocytes, menkel cells, vascular endothelial cells, hybridomas, and the like.

The medium used for the cell culture may be a liquid medium, a semisolid medium or a solid medium, which is employed in a usual cell culture, and can be selected in accordance with an object of the culture, the kind of the cells cultured, and the like.

Also, the culture vessel of the invention can be sterilized before or after the connection of each element. As the method for sterilization, there can be employed, for instance, gas sterilization by ethylene oxide gas; radiation sterilization by ultraviolet rays, gamma rays, X rays, or the like; or the like.

The culture vessel of the present invention will be more specifically described and explained by means of the following Examples, Comparative Examples and Test Examples, wherein all parts are by weight unless otherwise noted.

### Example 1

A body of the vessel 2 (diameter: 60 mm, height: 15 mm) was obtained by processing an acrylic resin. Then, as the monomer mixture for the gas permeable plastic material:

| | |
|---|---|
| tris(trimethylsiloxy)silylstyrene | 46 parts |
| 2,2,2,2',2',2'-hexafluoroisopropyl methacrylate | 54 parts |
| p-vinylbenzyl methacrylate | 6 parts |
| ethylene glycol dimethacrylate | 1 part |
| N-vinylpyrrolidone | 5 parts |
| methyl methacrylate | 4 parts |
| 2,2'-azobis-2,4-dimethylvaleronitrile (hereinafter referred to as V-65) | 0.1 part |

was mixed, dissolved and put into a test tube. The above monomer mixture was polymerized successively at 30°C for 16 hours, at 40°C for 24 hours and at 50°C for 8 hours in a constant-temperature water bath, and at 50°C for 5 hours in a circulating drier. The resulting mixture was subjected to a thermal polymerization with raising the temperature from 50° to 120°C at a rate of 10°C /1.5 hours and further subjected to the polymerization at 120°C for 1 hour, followed by cooling gradually to the room temperature to give the transparent gas permeable plastic material in the form of bar. The resulting gas permeable plastic material was cut into a film having a thickness of 0.4 mm and a diameter of 59 mm, and the obtained film was polished. The gas permeable plastic material film made was subjected to a plasma treatment at 50W for 3 minutes in oxygen atmosphere under the pressure of 0.8 torr.

Then, the gas permeable plastic material film was connected to the body of the vessel by a procedure comprising covering the bottom opening of the body of the vessel 2 with the gas permeable plastic material film 1 and fixing the gas permeable plastic material film 1 to the body using the bottom ring 4, and in a similar manner. as above the gas permeable plastic material film 1 was fixed to the body of the vessel 2 at the opposite side of the bottom ring 4 using the top ring 3, to complete the culture vessel shown in Fig. 3. The vessel made was sterilized using ethylene oxide gas, and subjected to aeration sufficiently.

### Example 2

The culture vessel shown in Fig. 3 was made in the same manner as in Example 1 by using the gas permeable plastic material which was made in the form of film having the thickness of 0.4 mm and the diameter of 59 mm and subjected to the plasma treatment, except that as the monomer mixture for the gas permeable plastic material:

| | |
|---|---|
| tris(trimethylsiloxy)silylstyrene | 100 parts |
| V-65 | 0.1 part |

was used.

### Example 3

The culture vessel shown in Fig. 3 was made in the same manner as in Example 1 by using the gas permeable plastic material which was made in the form of film having the thickness of 0.4 mm and the diameter of 59 mm and subjected to the plasma treatment, except that as the monomer mixture for the gas permeable plastic material:

| | |
|---|---|
| tris(trimethylsiloxy)silylpropyl methacrylate | 42.5 parts |
| trifluoromethyl methacrylate | 40 parts |
| methyl methacrylate | 5 parts |
| methacrylic acid | 5 parts |
| ethylene glycol dimethacrylate | 7.5 parts |
| V-65 | 0.1 part |

was used.

### Example 4

The culture vessel shown in Fig. 3 was made in the same manner as in Example 1 by using the gas permeable plastic material which was made in the form of film having the thickness of 0.4 mm and the diameter of 59 mm and subjected to the plasma treatment, except that as the monomer mixture for the gas permeable plastic material:

| | |
|---|---|
| tris(trimethylsiloxy)silylstyrene | 10 parts |
| tris(trimethylsiloxy)silylpropyl methacrylate | 10 parts |
| urethane bond-containing polysiloxane macromonomer represented by the formula (III) as shown below | 10 part |
| methyl methacrylate | 70 parts |
| V-65 | 0.1 part |

was used.

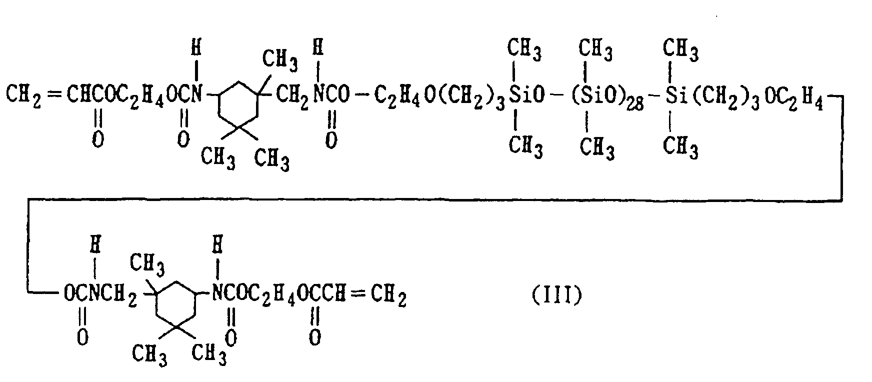

### Example 5

The culture vessel shown in Fig. 4 was made in the same manner as in Example 1 except that the unit for supplying a medium 9 was connected to the upper of the body of the vessel 2 made in Example 1 and the unit for discharging the medium and for collecting the supernatant of the medium or cells 10 was connected to the lower of the body of the vessel 2 (refer to Fig. 3).

### Comparative Example 1

The culture vessel shown in Fig. 3 was made in the same manner as in Example 1 except that the non-gas permeable plastic material was made by using

| | |
|---|---|
| methyl methacrylate | 97 parts |
| ethylene glycol dimethacrylate | 3 parts |
| AIBN | 0.1 part |

as the monomer mixture and the resulting non-gas permeable plastic material was used instead of the gas permeable plastic material.

Then, oxygen permeability and contact angle of test pieces made of the gas permeable plastic material films which were obtained in Examples 1 to 4 and the non-gas permeable plastic material film which was obtained in Comparative Example 1 were measured in accordance with the following methods. The results are shown in Table 1.

### (A) Oxygen permeability

Using a Seikaken-type film oxygen-gas permeater commercially available from RIKASEIKI KOGYO CO., LTD. (JAPAN), oxygen permeability of each test piece was measured in saline at 35°C. The unit of oxygen permeability is ml(O₂)·cm/(cm²·sec·mmHg).

### (B) Contact angle

Using a goniometer commercially available from ERMA OPTICAL WORKS LTD., contact angle (degree) was measured in accordance with cecil drop method at 25°C.

**Table 1**

| Ex. No. | Physical properties of test piece | |
|---|---|---|
| | Oxygen permeability (ml(O₂)·cm/(cm²·sec·mmHg)) | Contact angle (degree) |
| 1 | 250 × 10⁻¹¹ | 15 |
| 2 | 500 × 10⁻¹¹ | 60 |
| 3 | 100 × 10⁻¹¹ | 20 |
| 4 | 50 × 10⁻¹¹ | 20 |
| Com. Ex. 1 | 0.1 × 10⁻¹¹ | 20 |

As shown in Table 1, oxygen permeability was 250 × 10⁻¹¹ ml(O₂)·cm/(cm²·sec·mmHg), 500 × 10⁻¹¹ ml(O₂)·cm/(cm²·sec·mmHg), 100 x 10⁻¹¹ ml(O₂)·cm/(cm²·sec·mmHg) and 50 × 10⁻¹¹ ml(O₂)·cm/(cm²·sec·mmHg) in case of the test pieces obtained from films in Examples 1 to 4, respectively, while oxygen permeability was 0.1 × 10⁻¹¹ ml(O₂)·cm/(cm²·sec mmHg) in case of the test piece obtained from a film in Comparative Example 1. On the other hand, contact angle was 15 degrees, 60 degrees, 20 degrees and 20 degrees in case of the test pieces obtained from films in Examples 1 to 4, respectively, while contact angle was 20 degrees in case of the test piece obtained from a film in Comparative Example 1. From those results it can be understood that the gas permeable plastic materials made in Examples 1 to 4 are excellent as to oxygen permeability, and also excellent as to hydrophilic property.

### Test Example 1

### (1) Cell culture

A suspension of 3T3 cells (commercially available from DAINIPPON PHARMACEUTICAL CO., LTD., JAPAN) prepared using Dulbecco's modified Eagle's medium (hereinafter referred to as DMEM, commercially available from LIFE TECHNOLOGIES INC.) containing 10 % fetal bovine serum (hereinafter referred to as FBS) was seeded into each vessel prepared in Examples 1 to 3 by using the gas permeable plastic material film or into a vessel prepared in Comparative Example 1 by using the non-gas permeable plastic material film so as to result in 5 × 10³ cells/cm². Then, each vessel was filled with DMEM, and was hermetically sealed so that bubbles did not contaminate.

The resulting vessels were placed in a 5 % carbonic acid gas incubator, and the cultures were incubated at 37°C for 144 hours.

The 3T3 cells used in the Test Example 1 were obtained incubating the 3T3 cells in a usual vessel (catalog no. 178891, commercially available from Nalge Nunc International).

### (2) Collection of the cells

After removing the mediums in the vessels, the vessels were rinsed with Hank's solution, and trypsinization was carried out by adding dropwise 1 ml of a 0.25 w/v% trypsine solution (commercially available from Wako Pure Chemical Industries, Ltd., JAPAN) into the vessels. Then, the cultured cells were collected and suspended in DMEM containing 10 % FBS, and the number of living cells was counted by the dye-exclusion test using Trypan blue. The results are shown in Table 2.

**Table 2**

| Ex. No. | Number of living cells (cells/cm²) |
|---|---|
| 1 | 24 × 10⁴ |
| 2 | 35 × 10⁴ |
| 3 | 21 × 10⁴ |
| Com. Ex. 1 | 6 × 10⁴ |

As shown in Table 2, the numbers of living cells after the culture were 24 × 10⁴ cells/cm², 35 × 10⁴ cells/cm² and 21 × 10⁴ cells/cm² in case of the vessels made in Examples 1 to 3, respectively, while the number of living cells after the culture was 6 × 10⁴ cells/cm² in case of the vessel made in Comparative Example 1. From those results, it can be understood that the vessels made in Examples 1 to 3 are higher than one made in Comparative Example 1 in the number of living cells after the culture, and that the vessels made in Examples 1 to 3 provide an excellent condition for the culture.

In addition to the ingredients used in the Examples, other ingredients can be used as set forth in the specification to obtain substantially the same results.

A culture vessel is provided wherein at least a part of the vessel is made of a gas permeable plastic material, as defined in claim 1. In accordance with the present invention, the culture vessel can be constantly and sufficiently supplied with a gas such as oxygen necessary for metabolism of cells without an oxygenator, and the like, and is not contamined with bacteria and is easily transported.

## Claims

1. A culture vessel wherein at least a part of the vessel is made of a gas permeable plastic material having an oxygen permeability of at least 1 × 10⁻¹¹ ml O₂ · cm/(cm² · sec · mm Hg), wherein the gas permeable plastic material is obtainable by polymerizing a monomer mixture containing a silicon-containing monomer **characterized in that** said silicon-containing monomer is selected from the group consisting of a silicon-containing acrylate and/or methacrylate, a silicon-containing styrene derivative and a silicon-containing urethane macromonomer,
and the gas permeable plastic material is subjected to a plasma treatment to be rendered hydrophilic.

2. The culture vessel of claim 1, wherein the gas permeable plastic material is nonporous as a state where statistical void space (free volume) produced by movement (vibration) of high-molecular chains exists, but physically and spatially defined pores do not exist.

3. The culture vessel of claim 1 or 2, wherein the gas permeable plastic material has transparency.

4. The culture vessel of claim 1, wherein the silicon-containing acrylate and/or methacrylate is represented by the formula (I): 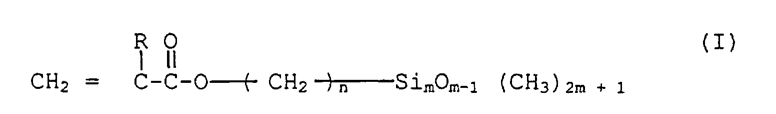 wherein R is a hydrogen atom or a methyl group, n is an integer of 1 to 3 and m is an integer of 1 to 4.

5. The culture vessel of claim 1, wherein the silicon-containing styrene derivative is represented by the formula (II): 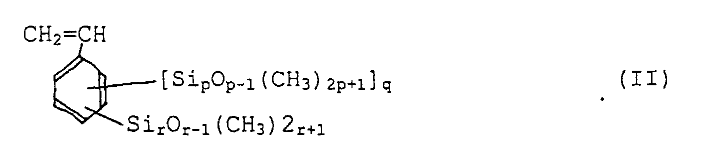 wherein p is an integer of 1 to 15, q is 0 or 1, and r is an integer of 1 to 15, with the proviso that a case of q=0 and r=1 is excepted.

6. The culture vessel of claim 1, wherein the silicon-containing urethane macromonomer is represented by the formula:
A¹-U¹-S¹-U²-A²
wherein A¹ is a group represented by the formula:
Y²¹-R³¹-
In which Y²¹ is an acryloyloxy group, a methacryloyloxy group, a vinyl group or an allyl group and R³¹ is a straight-chain or branched alkylene group having 2 to 6 carbon atoms; A² is a group represented by the formula
-R³⁴-Y²²
in which Y²² is an acryloyloxy group, a methacryloyloxy group, a vinyl group or an allyl group and R³⁴ is a straight-chain or branched alkylene group having 2 to 6 carbon atoms; U¹ is a group represented by the formula:
-X²¹-E²¹-X²⁵-R³²-
in which X²¹ is a covalent bond, oxygen atom or an alkylene glycol group having 1 to 6 carbon atoms, E²¹ is a -CONH-group or a divalent group derived from a diisocyanate selected from a group consisting of a saturated or unsaturated aliphatic diisocyanate, an alicyclic diisocyanate and an aromatic diisocyanate, X²⁵ is an oxygen atom or an alkylene glycol group having 1 to 6 carbon atoms, and R³² is a straight-chain or branched alkylene group having 1 to 6 carbon atoms; S¹ is a group represented by the formula: 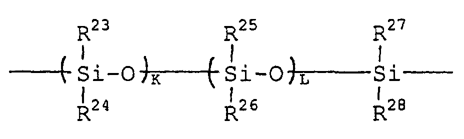 in which each of R²³, R²⁴, R²⁵, R²⁶, R²⁷ and R²⁸ is independently an alkyl group having 1 to 6 carbon atoms, an alkyl group substituted with fluorine or phenyl group, K is an integer of 1 to 50, L is an integer of 0 to (50-K); and
U² is a group represented by the formula:
-R³³-X²⁶-E²²-X²²-
in which R³³ is a straight-chain or branched alkylene group having 1 to 6 carbon atoms, X²² is a covalent bond, oxygen atom or an alkylene glycol group having 1 to 6 carbon atoms, X²⁶ is an oxygen atom or an alkylene glycol group having 1 to 6 carbon atoms, and E²² is a -CONH- group or a divalent group derived from a diisocyanate selected from a group consisting of a saturated or unsaturated aliphatic diisocyanate, an alicyclic diisocyanate and an aromatic diisocyanate.

7. The culture vessel of any of claims 1 to 6, wherein cultured cells adhere on a surface of the gas permeable plastic material thereof.

8. The culture vessel of any of claims 1 to 7, wherein the culture vessel is a hermetic structure which allows sufficient aeration necessary for carrying out the usual cell culture, but does not allow seepage of an inside liquid of the vessel and does not allow contamination by an outside liquid of the vessel.

9. The culture vessel of any of claims 1 to 8, excepting the culture vessel wherein a bottom surface is a curved surface.

10. The culture vessel of any of claims 1 to 9, wherein a supply port is provided in at least a part of the culture vessel.

11. The culture vessel of any of claims 1 to 10, wherein a discharge port is provided in at least a part of the culture vessel.

## Patentansprüche

1. Kulturgefäß, bei dem mindestens ein Teil des Gefäßes aus einen gasdurchlässigen Kunststoffmaterial hergestellt ist, welches eine Sauerstoffdurchlässigkeit von mindestens (1x10⁻¹¹ ml O₂ · cm/ (cm² · s· mmHg) aufweist und das gasdurchlässige Kunststoffmaterial erhältlich ist, indem eine Monomermischung, die ein Silicium enthaltendes Monomer enthält, polymerisiert wird, **dadurch gekennzeichnet, dass** das Silicium enthaltende Monomer aus der Gruppe gewählt ist, die aus einem Silicium enthaltenden Acrylat und/oder Methacrylat, einem Silicium enthaltenden Styrolderivat und einem Silicium enthaltenden Urethanmakromonomer besteht, und das gasdurchlässige Kunststoffmaterial einer Plasmabehandlung unterworfen worden ist, um es hydrophil zu machen.

2. Kulturgefäß nach Anspruch 1, worin das gasdurchlässige Kunststoffmaterial als Zustand nicht porös ist, bei dem ein statistischer Hohlraum (freies Volumen), der durch die Bewegung (Schwingung) von hoch molekularen Ketten erzeugt wird, vorhanden ist, jedoch physikalisch und räumlich definierte Poren nicht existieren.

3. Kulturgefäß nach Anspruch 1 oder 2, worin das gasdurchlässige Kunststoffmaterial eine Transparenz besitzt.

4. Kulturgefäß nach Anspruch 1, worin das Silicium enthaltende Acrylat und/oder Methacrylat durch die Formel (I) dargestellt ist: 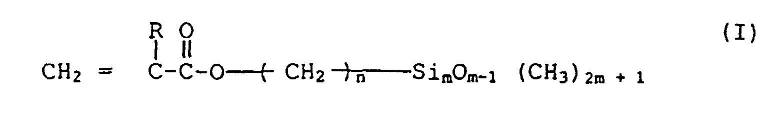 worin R ein Wasserstoffatom oder eine Methylgruppe ist, n eine ganze Zahl von 1 bis 3 bedeutet und m eine Zahl von 1 bis 4 darstellt.

5. Kulturgefäß nach Anspruch 1, worin das Silicium enthaltende Styrolderivat durch die Formel (II) dargestellt ist: 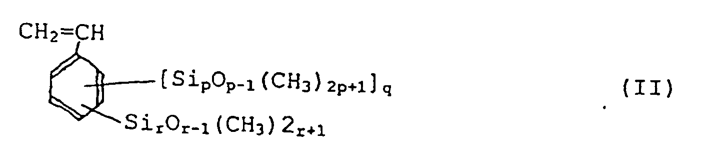 worin p eine ganze Zahl von 1 bis 15 ist, q 0 oder 1 bedeutet und r eine Zahl von 1 bis 15 darstellt, unter der Voraussetzung, dass der Fall q = 0 und r = 1 erwartet wird.

6. Kulturgefäß nach Anspruch 1, worin das Silicium enthaltende Urethanmakromonomer durch die Formel
A¹-U¹-S¹-U²-A²
dargestellt ist, worin A¹ eine Gruppe darstellt, die durch die Formel
Y²¹-R³¹-
dargestellt ist, worin Y²¹ eine Acryloyloxygruppe, eine Methacryloyloxygruppe, eine Vinylgruppe oder eine Allylgruppe darstellt und R³¹ eine geradkettige oder verzweigte Alkylengruppe mit 2 bis 6 Kohlenstoffatomen bedeutet; A² eine Gruppe ist, die durch die Formel
-R³⁴-X²²
dargestellt ist, worin Y²² eine Acryloyloxygruppe, eine Methacryloyloxygruppe, eine Vinylgruppe oder eine Allylgruppe ist und R³⁴ eine geradkettige oder verzweigte Alkylengruppe mit 2 bis 6 Kohlenstoffatomen bedeutet; U¹ eine Gruppe ist, die durch die Formel:
-X²¹-E²¹-X²⁵-R³²-
dargestellt ist, worin X²¹ eine kovalente Bindung, ein Sauerstoffatom oder eine Alkylenglykolgruppe mit 1 bis 6 Kohlenstoffatomen bedeutet, E²¹ eine -CONH-Gruppe oder eine zweiwertige Gruppe von einem Diisocyanat bedeutet, das aus der Gruppe gewählt ist, die aus einem gesättigten oder ungesättigten aliphatischen Diisocyanat, einem alicyclischen Diisocyanat und einem aromatischen Diisocyanat gewählt ist, X²⁵ ein Sauerstoffatom oder eine Alkylenglykolgruppe mit 1 bis 6 Kohlenstoffatomen bedeutet und R³² eine geradkettige oder verzweigte Alkylengruppe mit 1 bis 6 Kohlenstoffatomen darstellt; S¹ eine Gruppe ist, die durch die Formel: 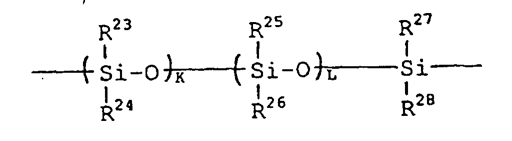 dargestellt ist, worin jeweils voneinander unabhängig R²³, R²⁴, R²⁵, R²⁶, R²⁷ and R²⁸ eine Alkylgruppe mit 1 bis 6 Kohlenstoffatomen, eine Alkylgruppe, die mit einem Fluor oder einer Phenylgruppe substituiert ist, bedeuten, K eine ganze Zahl von 1 bis 50 darstellt, L eine ganze Zahl von 0 bis (50-K) bedeutet und U² eine Gruppe ist, die durch die Formel
-R³³-X²⁶-E²²-X²²-
dargestellt ist, worin R³³ eine geradkettige oder verzweigte Alkylengruppe mit 1 bis 6 Kohlenstoffatomen bedeutet, X²² eine kovalente Bindung, ein Sauerstoffatom oder eine Alkylenglykolgruppe mit 1 bis 6 Kohlenstoffatomen darstellt, X²⁶ ein Sauerstoffatom oder eine Alkylenglykolgruppe mit 1 bis 6 Kohlenstoffatomen darstellt und E²² eine -CONH-Gruppe oder eine zweiwertige Gruppe von einem Diisocyanat ist, das aus der Gruppe gewählt ist, die aus einem gesättigten oder ungesättigten aliphatischen Diisocyanat, einem alicyclischen Diisocyanat und einem aromatischen Diisocyanat besteht.

7. Kulturgefäß nach einem der Ansprüche 1 bis 6, worin die kultivierten Zellen an einer Oberfläche des gasdurchlässigen Kunststoffmaterials haften.

8. Kulturgefäß nach einem der Ansprüche 1 bis 7, worin das Kulturgefäß eine abgeschlossene Struktur ist, die eine ausreichende Belüftung, die zur Durchführung einer üblichen Zellkultivierung notwendig ist, ermöglicht, wobei allerdings nicht das Durchsickern der im Innern des Gefäßes befindlichen Flüssigkeit möglich ist und ebenfalls nicht eine Kontamination durch eine von außen kommende Flüssigkeit in das Gefäß möglich ist.

9. Kulturgefäß nach einem der Ansprüche 1 bis 8, wobei ein Kulturgefäß ausgeschlossen ist, worin die Bodenoberfläche eine gekrümmte Oberfläche ist.

10. Kulturgefäß nach einem der Ansprüche 1 bis 9, worin eine Zufuhröffnung an mindestens einem Teil des Kulturgefäßes vorgesehen ist.

11. Kulturgefäß nach einem der Ansprüche 1 bis 10, worin eine Auslaßöffnung an mindestens einem Teil des Kulturgefäßes vorgesehen ist.

## Revendications

1. Récipient de culture dans lequel au moins une partie du récipient est faite en une matière plastique perméable aux gaz ayant une perméabilité à l'oxygène d'au moins 1 x 10⁻¹¹ ml O₂. cm/(cm²·s·mm de Hg), dans lequel la matière plastique perméable aux gaz peut être obtenue par polymérisation d'un mélange de monomères contenant un monomère contenant du silicium, **caractérisé en ce que** ledit monomère contenant du silicium est choisi dans le groupe constitué par un acrylate et/ou méthacrylate contenant du silicium, un dérivé de styrène contenant du silicium et un macromonomère d'uréthane contenant du silicium, et **en ce qu'**on soumet la matière plastique perméable aux gaz à un traitement par plasma pour la rendre hydrophile.

2. Récipient de culture selon la revendication 1, **caractérisé en ce que** la matière plastique perméable aux gaz est non poreuse dans un état où l'espace de vide statistique (volume libre) produit par le mouvement (la vibration) de chaînes de masse moléculaire élevée existe, mais où des pores définis physiquement et spatialement n'existent pas.

3. Récipient de culture selon la revendication 1 ou 2, **caractérisé en ce que** la matière plastique perméable aux gaz est transparente.

4. Récipient de culture selon la revendication 1, **caractérisé en ce que** l'acrylate et/ou le méthacrylate contenant du silicium est représenté par la formule (I): 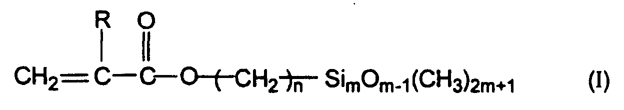 dans laquelle R est un atome d'hydrogène ou un groupe méthyle, n est un nombre entier de 1 à 3 et m est un nombre entier de 1 à 4.

5. Récipient de culture selon la revendication 1, **caractérisé en ce que** le dérivé de styrène contenant du silicium est représenté par la formule (II) : 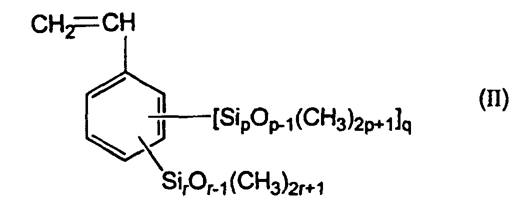 dans laquelle p est un nombre entier de 1 à 15, q vaut 0 ou 1 et r est un entier de 1 à 15, sous réserve que le cas où q=0 et r=1 soit exclu.

6. Récipient de culture selon la revendication 1, **caractérisé en ce que** le macromonomére d'uréthane contenant du silicium est représenté par la formule :
A¹-U¹-S¹-U²-A²
dans laquelle A¹ est un groupe représenté par la formule :
Y²¹ - R³¹ -
dans laquelle Y²¹ est un groupe acryloyloxy, un groupe méthacryloyloxy, un groupe vinyle ou un groupe allyle et R³¹ est un groupe alkylène à chaîne linéaire ou ramifiée ayant 2 à 6 atomes de carbone ;
A² est un groupe représenté par la formule :
- R³⁴ - Y²²
dans laquelle Y²² est un groupe acryloyloxy, un groupe méthacryloyloxy, un groupe vinyle ou un groupe allyle et R³⁴ est un groupe alkylène à chaîne linéaire ou ramifiée ayant 2 à 6 atomes de carbone ;
U¹ est un groupe représenté par la formule :
- X²¹ - E²¹ - X²⁵ - R³² -
dans laquelle X²¹ est une liaison covalente, un atome d'oxygène ou un groupe alkyléneglycol ayant 1 à 6 atomes de carbone, E²¹ est un groupe -CONH- ou un groupe divalent dérivé d'un diisocyanate choisi dans un groupe constifué d'un diisocyanate aliphatique saturé ou insaturé, d'un diisocyanate alicyclique et d'un diisocyanate aromatique, X²⁵, est un atome d'oxygène ou un groupe alkyléneglycol ayant 1 à 6 atomes de carbone, et
R³² est un groupe alkyléne à chaîne linéaire ou ramifiée ayant 1 à 6 atomes de carbone ;
S¹ est un groupe représenté par la formule : 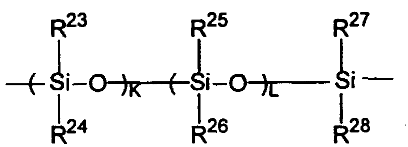 dans laquelle chaque R²³, R²⁴, R²⁵, R²⁶, R²⁷ et R²⁸ est indépendamment un groupe alkyle ayant 1 à 6 atomes de carbone, un groupe alkyle substitué par du fluor ou un groupe phényle, K est un nombre entier de 1 à 50, L est un nombre entier de 0 à (50-K) ; et
U² est un groupe représenté par la formule :
- R³³ - X²⁶ - E²² - X²² -
dans laquelle R³³ est un groupe alkylène à chaîne linéaire ou ramifiée ayant 1 à 6 atomes de carbone, X²² est une liaison covalente, un atome d'oxygène ou un groupe alkyléneglycol ayant 1 à 6 atomes de carbone,
X²⁶ est un atome d'oxygène ou un groupe alkylèneglycol ayant 1 à 6 atomes de carbone, et E²² est un groupe -CONH- ou un groupe divalent dérivé d'un diisocyanate choisi dans le groupe constitué par un diisocyanate aliphatique saturé ou insaturé, un diisocyanate alicyclique et un diisocyanate aromatique.

7. Récipient de culture selon l'une quelconque des revendications 1 à 6, **caractérisé en ce que** les cellules cultivées adhèrent à une surface de matière plastique perméable aux gaz de celui-ci.

8. Récipient de culture selon l'une quelconque des revendications 1 à 7, **caractérisé en ce que** le récipient de culture est une structure hermétique qui permet une aération suffisante nécessaire pour effectuer la culture de cellules classique, mais ne permet pas l'infiltration d'un liquide à l'intérieur du récipient et ne permet pas la contamination par un liquide extérieur au récipient.

9. Récipient de culture selon Tune quelconque des revendications 1 à 8, à l'exception d'un récipient de culture dans lequel la surface du fond est une surface incurvée.

10. Récipient de culture selon Tune quelconque des revendications 1 à 9, **caractérisé en ce qu'**un orifice d'amenée est prévu sur au moins une partie du récipient de culture.

11. Récipient de culture selon l'une quelconque des revendications 1 à 10, **caractérisé en ce qu'**un orifice de décharge est prévu sur au moins une partie du récipient de culture.
